Europäisches Patentamt

European Patent Office

Office européen des brevets

(19)

(11) Veröffentlichungsnummer: **0 212 537 B1**

(12) **EUROPÄISCHE PATENTSCHRIFT**

(45) Veröffentlichungstag der Patentschrift:
03.01.90

(51) Int. Cl.⁴: **C07D 213/74, A61K 31/44**

(21) Anmeldenummer: **86111118.5**

(22) Anmeldetag: **12.08.86**

(54) Verfahren zur Herstellung einer stabilen Modifikation von Torasemid sowie Arzneimittel enthaltend Torasemid.

(30) Priorität: **17.08.85 DE 3529529**

(43) Veröffentlichungstag der Anmeldung:
**04.03.87 Patentblatt 87/10**

(45) Bekanntmachung des Hinweises auf die Patenterteilung:
**03.01.90 Patentblatt 90/1**

(84) Benannte Vertragsstaaten:
**AT BE CH DE FR GB IT LI LU NL SE**

(56) Entgegenhaltungen:
**EP-A- 0 003 383**
**DE-A- 2 516 025**

CHEMICAL ABSTRACTS, Band 88, Nr. 25, 19. Juni 1978, - Columbus, Ohio, USA L.DUPONT et al. "Crystal and molecular structure of a diuretic..." Seite 686, Spalte 2, Zusammenfassung-Nr. 201 423f
CHEMICAL ABSTRACTS, Band 89, Nr. 18, 30. Oktober 1978, Columbus, Ohio, USA L.DUPONT et al. "Structure of a second form of torasemide" Seite 600, Spalte 2, Zusammenfassung-Nr. 155 867z

(73) Patentinhaber: **BOEHRINGER MANNHEIM GMBH, Sandhofer Strasse 116, D-6800 Mannheim 31(DE)**

(72) Erfinder: **Topfmeier, Fritz, Dr. rer. nat., Zeppelinstrasse 157, D-6900 Heidelberg(DE)**
Erfinder: **Lettenbauer, Gustav, Dr. rer. nat., Am Weingarten 1, D-6840 Lampertheim(DE)**

## Beschreibung

Gegenstand der vorliegenden Erfindung ist ein Verfahren zur Herstellung einer stabilen Modifikation von Torasemid.

Torasemid (1-Isopropyl-3-[(4-m-toluidino-3-pyridyl)sulfonyl]-harnstoff ist eine aus der DE-OS 25 16 025, Beispiel 71 bekannte Verbindung mit interessanten pharmakologischen Eigenschaften. Insbesondere wirkt diese Verbindung stark diuretisch, wobei Wasser und Natriumionen relativ stärker ausgeschieden werden als Kaliumionen. Die Verbindung ist deshalb als Diuretikum von großem Interesse.

Bei der Herstellung dieser Verbindung wird normalerweise eine Reinigung angeschlossen, bei der die betreffende Substanz in einer wässrigen oder wässrig alkoholischen Lösung von Natriumhydrogencarbonat gelöst und nach Abfiltrieren von Verunreinigungen z.B. mit Essigsäure oder $CO_2$ als Torasemid wieder abgeschieden wird. Das Produkt fällt bei diesem Verfahren in weißen Kristallen mit einem Schmelzpunkt von 163 - 164°C an.

Aus Acta Cryst. 1978, Seite 2659 - 2662 und Acta Cryst. 1978, Seite 1304 - 1310 ist bekannt, daß Torasemid in zwei Modifikation vorkommen kann, die sich röntgen-kristallographisch unterscheiden. Beide Modifikationen entstehen nebeneinander, wenn man eine Lösung von Torasemid in Petrolether/Ethanol langsam eindampft. Die Kristalle, die sowohl als Prismen mit einem Schmelzpunkt von 169°C als auch als Blättchen mit einem Schmelzpunkt von 162°C charakterisiert werden, sind in dieser Literaturstelle jedoch nur bezüglich ihrer röntgen-kristallographischen Eigenschaften beschrieben worden. Die Modifikation mit dem Schmelzpunkt von 169°C, die im folgenden als Modifikation I bezeichnet wird, kristallisiert monoclin in der Raumgruppe P2₁/c, die mit dem Schmelzpunkt von 162 °C, die im folgenden als Modifikation II bezeichnet wird, kristallisiert monoclin in der Raumgruppe P2/n.

Die bei der Herstellung und normalen Reinigung durch Umfällung von Torasemid mit $CO_2$ anfallende Modifikation ist die Modifikation II, welche üblicherweise auch bei Umkristallisieren aus anderen Lösungsmitteln entsteht. Da diese Form sich bei der Lagerung des reinen Wirkstoffes nicht verändert und bei allen Reinigungsversuchen die vorherrschende Form bildet, wurde angenommen, daß diese Modifikation II auch beständig ist. Überraschenderweise wurde nun festgestellt, daß sich Torasemid der Modifikation II, wenn es in feinster Verteilung in einer pharmazeutischen Tablette vorliegt, mehr oder weniger schnell in die Modifikation I umlagert, wodurch sich Kristallgröße und Lösungsgeschwindigkeit des Wirkstoffes beim Einbringen der Tablette in Wasser signifikant verändern können. Da andererseits die Lösungsgeschwindigkeit bekanntermaßen eine der wichtigen Kenngrößen einer pharmazeutischen Applikationsform darstellt und diese, um reproduzierbar dosieren zu können, sich von Tablette zu Tablette nicht unterscheiden darf, stellte sich die Aufgabe, eine Torasemidapplikationsform zu finden, welche sich in ihrer Lösungsgeschwindigkeit während der Lagerung nicht verändert. Da die unkontrollierbare Änderung der Lösungsgeschwindigkeit auf der Umlagerung der Modifikation II in die Modifikation I des Torasemids beruht, lag es nahe, von vornherein die Modifikation I einzusetzen, von der aus unseren Versuchen hervorging, daß sie auch in der Tablette stabil ist und sich nicht wieder zurück in die Modifikation II umlagert. Gegenstand der Erfindung sind daher auch orale Darreichungsformen, die Torasemid der Modifikation I als Wirkstoff enthalten.

Das für die Röntgen-Kristallographie ausreichende Verfahren, beide Modifikationen nebeneinander aus dem gleichen Lösungsgemisch auskristallisieren zu lassen und nach ihrer makroskopischen Kristalltracht von Hand zu verlesen, ist natürlich für eine technische Fertigung unbrauchbar, da eine entsprechende Separierung der Kristalle nicht möglich ist. Darüber hinaus war bekannt, daß Torasemid beim Erhitzen in den meisten Lösungsmitteln irreversibel mit dem Anilinostickstoffatom cyclisiert. Eine Umkristallisation aus den meisten Lösungsmitteln ist deshalb zur Herstellung der Modifikation I nicht geeignet. Es stellte sich daher die weitere Aufgabe, ein Verfahren zur Herstellung der reinen Modifikation I des Torasemids zu finden, welches einfach und wirtschaftlich durchzuführen ist und ohne Zersetzung des Torasemids verläuft.

Überraschenderweise wurde gefunden, daß man Torasemid der Modifikation II dadurch in die Modifikation I umlagern kann, daß man eine Suspension derselben in Wasser mit fein verteilten Kristallkeimen der Modifikation I animpft und diese Suspension so lange rührt, bis sich die gesamte Menge in die Modifikation I umgelagert hat.

Bei Raumtemperatur verläuft diese Umlagerung relativ langsam, so daß Zeiten von 10 - 14 Tagen notwendig sind. Die Suspension kann jedoch auch auf Temperaturen von 70 - 90°C erwärmt werden, wobei die Reaktion in 3- 6 Stunden vollständig abläuft. Während beim Erhitzen von Torasemid in Lösungsmitteln wie Äthanol, Essigester, Methylenchlorid und Chloroform in erheblichen Mengen Zersetzungsprodukte entstehen, kann Torasemid überraschenderweise in Wasser ohne merkliche Zersetzung mehrere Tage auf 90°C erhitzt werden. Weiterhin erscheint es überraschend, daß bereits die Zugabe von geringen Mengen (bis 1 %) eine Umlagerung bewirken, da Torasemid der Modifikation I immerhin eine Löslichkeit von 1,9 g/l bei 90°C besitzt. Rein aufgrund der Löslichkeit wäre zu erwarten gewesen, daß sich diese geringe Menge von Torasemid in dem Suspensionsmedium Wasser auflöst.

Da die Umlagerung selbst in reinem Wasser abläuft, hat dieses Verfahren den weiteren Vorteil, daß keine zusätzlichen Verunreinigungen wie Lösungsmittel, katalytisch wirkende Säuren oder Basen etc. in das Produkt eingeschleppt werden, im Gegenteil die durch den Rekristallisationsvorgang in der ursprünglichen Modifikation II enthaltenen Verunreinigungen in das Wasser abgegeben werden.

Orale Darreichungsformen mit Torasemid der Modifikation I werden in üblicher Weise hergestellt unter Verwendung pharmakologisch verträglicher Hilfsstoffe, wie z.B. Zucker, Stärke, Stärkederivate, Cellulose, Cellulosederivate, Formentrennmittel, Antiklebemittel sowie ggf. Fließregulierungsmittel. Insbesondere können beim Einsatz von Torasemid der Modifikation I wäßrige Verfahrensschritte z. B. Granulation durchgeführt werden.

Für die angestrebten Qualitätsbestimmenden Parameter der erfinderischen Darreichungsform ist est besonders vorteilhaft, wenn der Wirkstoff Torasemid Modifikation I mit folgender Teilchengröße-Verteilung eingesetzt wird:
mindestens 90 % ≤ 96 μm und
mindestens 50 % ≤ 48 μm.

Im Vergleich zu pharmazeutischen Formulierungen mit dem Wirkstoff Torasemid Modifikation II haben die erfinderischen Formulierungen eine auch nach längerer Lagerung bei über Raumtemperatur liegenden Temperaturen und höheren relativen Luftfeuchten gleichbleibend rasche in-vitro-Auflösungsraten.

Die rasch einsetzende pharmakologische Wirkung dieser Zubereitungen wird durch die schnelle Auflösungsgeschwindigkeit ·des Wirkstoffes aus der Darreichungsform gewährleistet. So sind z. B. nach 15 Minuten mehr als 60 %, nach 30 Minuten mehr als 80 % in Lösung gegangen. (Testmethode: Paddle-Test USP XXI)

In den folgenden Beispielen ist die Erfindung näher erläutert.

Beispiel 1:

10 kg Torasemid, welches gemäß DE-OS 25 16 025 hergestellt und durch Umfällen aus Natriumdicarbonatlösung mit $CO_2$ gereinigt wurde, werden in der 10fachen Menge Wasser suspendiert und 100 g Torasemid Modifikation I aus einem früheren Ansatz zugefügt. Die Suspension wird auf 90°C erhitzt, bei dieser Temperatur 6 Stunden gerührt und wieder auf Raumtemperatur abgekühlt und nochmals 30 Minuten nachgerührt. Danach wird das Kristallisat abgesaugt, mit 40 Liter Wasser nachgewaschen und im Vakuumschrank bei 50°C getrocknet. Es werden 9,91 kg Torasemid der Modifikation I erhalten.

Das Röntgenbeugungsdiagramm entspricht der reinen Modifikation I, eine Reinheitsprüfung mit HPLC entspricht der reinen Ausgangssubstanz.

Kristallkeime der Modifikation I können ggf. auch nach dem in Acta Cryst. 1978, S. 1304 beschriebenen Verfahren erhalten werden.

Beispiel 2:

900 g Torasemid Modifikation II werden in 10 Liter Wasser suspendiert und in Gegenwart von 10 g Torasemid Modifikation I bei Raumtemperatur gerührt. Nach 8 Tagen weist eine entnommene Probe keine Spur von Torasemid Modifikation II mehr auf. Das Produkt wird abfiltriert und im Vakuumtrockenschrank bei 50°C getrocknet, wodurch 875 g Torasemid Modifikation I erhalten werden. Die Reinheit entspricht der Ausgangssubstanz, das röntgenkristallographische Spektrum der reinen Modifikation I.

Zum Vergleich wurde der gleiche Ansatz jedoch ohne Zusatz der Modifikation I bei Raumtemperatur 10 Tage lang gerührt, ohne daß eine Umlagerung in die Modifikation I eintrat.

Beispiel 3:

10 kg rohes Torasemid, welches gemäß der Vorschrift der DE-OS 25 16 025 hergestellt wurde, wird in 100 Liter Wasser suspendiert und mit 30 Liter 1 N Natronlauge versetzt. Es entsteht nach Behandlung mit 500 g Aktivkohle und Abfiltrieren eine klare gelbliche Lösung, aus der durch Zugabe von 1 N Schwefelsäure bei Raumtemperatur bis zum Erreichen eines pH-Werts von 7,5 das Torasemid wieder ausgefällt wird (Verbrauch ca. 29 Liter). Zu dieser Suspension werden 100 g Torasemid Modifikation I zugegeben und die Lösung 6 Stunden auf 90°C erhitzt. Während dieser Zeit tritt die Modifikationsumlagerung ein. Es wird auf Raumtemperatur abgekühlt und das Kristallisat abzentrifugiert. Das Kristallisat wird mit 50 Liter Wasser nachgewaschen und anschließend bei 50°C im Vakuumschrank getrocknet. Ausbeute 9,82 kg reines Torasemid der Modifikation I.

Dieses Beispiel zeigt, daß die erfindungsgemäße Umlagerung auch in Anwesenheit von Fremdsalzen, wie sie bei der normalen Ausfällung des Torasemids vorliegen, durchgeführt werden kann.

Beispiel 4:

Herstellung einer 2,5 mg Tablette.

Torasemid der Modifikation I wird mit Lactose x $1H_2O$ und Maisstärke in üblicher Weise gemischt, mit Wasser granuliert, getrocknet und gesiebt (Granulat 1). Hochdisperses Siliciumdioxid und Magnesiumstearat werden gemischt, gesiebt und mit Granulat 1 gemischt. Diese Mischung wird in üblicher Weise tablettiert.

Ansatz für 100.000 Tabletten.

| Herstellungsformel für 100 000 Tabletten | |
| --- | --- |
| Torasemid | 0,25 kg |
| Lactose · $1H_2O$ | 6,05 kg |
| Maisstärke | 1,60 kg |
| Siliciumdioxid, hochdispers | 60,00 g |
| Magnesiumstearat | 40,00 g |
| Wasser, gereinigt | 1,20 kg |

Beispiel 5:

Herstellung einer 100 mg Tablette.

Torasemid der Modifikation I wird mit Lactose x $1H_2O$, Maisstärke und einer Teilmenge Magnesiumstearat vermischt. Die Mischung wird kompaktiert und auf die gewünschte Korngröße und Korngrößenverteilung gesiebt (Granulat 1). Hochdisperses Siliciumdioxid und Magnesiumstearat werden gemischt und gesiebt und mit dem Granulat 1 gemischt. Diese Mischung wird in üblicher Weise tablettiert.

Ansatz für 100.000 Tabletten.

| Herstellungsformel für 100 000 Tabletten | |
|---|---|
| Torasemid | 10,0 kg |
| Lactose · 1H₂O | 2,0 kg |
| Maisstärke | 7,7 kg |
| Siliciumdioxid, hochdispers | 0,2 kg |
| Magnesiumstearat | 0,1 kg |

**Patentansprüche**

1. Verfahren zur Herstellung von kristallinem Torasemid in der reinen Modifikation I (monoklin, Raumgruppe P2₁/c, Schmelzpunkt 169°C aus Torasemid der Modifikation II (monoklin, Raumgruppe P2/n, Schmelzpunkt 162°C), dadurch gekennzeichnet, daß man eine Suspension von Torasemid der Modifikation II in Wasser unter Zusatz von katalytischen Mengen der Modifikation I rührt, bis die Umlagerung beendet ist.

2. Verfahren nach Anspruch 1, dadurch gekennzeichnet, daß die Umlagerung bei Temperaturen zwischen Raumtemperatur und 90°C durchgeführt wird und die Umlagerungszeit 14 Tage bis 3 Stunden beträgt.

3. Verfahren gemäß Anspruch 1 oder 2, dadurch gekennzeichnet, daß Torasemid der Modifikation II in Form der beim Ausfällen von Torasemid aus alkalischer Lösung anfallenden, salzhaltigen, etwa neutralen Lösung eingesetzt wird.

4. Pharmazeutische Präparate enthaltend Torasemid und pharmakologisch verträgliche Trägerstoffe, dadurch gekennzeichnet, daß der Wirkstoff in der Modifikation I vorliegt.

5. Pharmazeutische Präparate, hergestellt aus kristallinem Torasemid in der reinen Modifikation I (monoklin, Raumgruppe P2₁/c, Schmelzpunkt 169°C) und pharmakologisch verträglichen Trägerstoffen.

6. Pharmazeutische Präparate gemäß Anspruch 4 oder 5, dadurch gekennzeichnet, daß die Auflösungsrate des Wirkstoffes Torasemid mindestens 60% nach 15 Minuten beträgt.

7. Pharmazeutische Präparate gemäß Anspruch 4 oder 5, dadurch gekennzeichnet, daß die Auflösungsrate des Wirkstoffes Torasemid mindestens 80% nach 30 Minuten beträgt.

**Claims**

1. Process for the preparation of crystalline torasemide in the pure modification I (monoclinic, space group P2₁/c, melting point 169°C.) from torasemind of the modification II (monoclinic, space group P2/n, melting point 162°C.), characterised in that one stirs a suspension of torasemide of the modification II in water with the addition of catalytic amounts of modification I until the rearrangement is ended.

2. Process according to claim 1, characterised in that the rearrangement is carried out at temperatures between room temperature and 90°C. and the rearrangement time amounts to 14 days to 3 hours.

3. Process according to claim 1 or 2, characterised in that torasemide of the modification II is used in the form of the salt-containing, approximately neutral solution obtained by the precipitation of torasemide from alkaline solution.

4. Pharmaceutical compositions containing torasemide and pharmacologically acceptable carriers, characterised in that the active material is present in modification I.

5. Pharmaceutical compositions prepared from crystalline torasemide in the pure modification I (monoclinic, space group P2₁/c, melting point 169°C.) and pharmacologically acceptable carriers.

6. Pharmaceutical compositions according to claim 4 or 5, characterised in that the dissolving rate of the active material torasemide amounts to at least 60% after 15 minutes.

7. Pharmaceutical compositions according to claim 4 or 5, characterised in that the dissolving rate of the active material torasemide amounts to at least 80% after 30 minutes.

**Revendications**

1. Procédé de préparation de la variété I pure du torasemide cristallisé (monoclinique, groupe P2₁/c, point de fusion 169°C), à partir de la variété II du torasemide (monoclinique, groupe P2/n, point de fusion 162°C), caractérisé en ce que l'on agite une suspension de la variété II torasemide dans l'eau, avec addition de quantités catalytiques de la variété I, jusqu'à la fin de la transformation.

2. Procédé selon la revendication 1, caractérisé en ce que la transformation est effectuée à des températures comprises entre la température ambiante et 90°C et que le temps de transformation est de 14 jours à 3 heures.

3. Procédé selon la revendication 1 ou 2, caractérisé en que la variété II du torasemide est mise en œuvre sous la forme de la solution environ neutre, saline, obtenue lors de la précipitation du torasemide à partir d'une solution alcaline.

4. Préparation pharmaceutique contenant du torasemide et des substances de support pharmacologiquement acceptables, caractérisée en ce que la substance active est sous la forme de la variété I.

5. Préparation pharmaceutique, préparée à partir de la variété I pure du torasemide cristallisé (monoclinique, groupe P2₁/c, point de fusion 169°C), et de substances de support pharmacologiquement acceptables.

6. Préparation pharmaceutique selon la revendication 4 ou 5, caractérisée en ce que le taux de dissolution de la substance active torasemide est d'au moins 60% au bout de 15 minutes.

7. Préparation pharmaceutique selon la revendication 4 ou 5, caractérisée en ce que le taux de dissolution de la substance active torasemide est d'au moins 80% au bout de 30 minutes.